(19) Europäisches Patentamt
European Patent Office
Office européen des brevets

(11) **EP 2 783 629 A1**

(12) **DEMANDE DE BREVET EUROPEEN**

(43) Date de publication:
**01.10.2014 Bulletin 2014/40**

(51) Int Cl.:
**A61B 5/08** (2006.01)   **A61B 7/00** (2006.01)

(21) Numéro de dépôt: **14305455.9**

(22) Date de dépôt: **28.03.2014**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**

(30) Priorité: **29.03.2013 FR 1352868**

(71) Demandeur: **Nutral SAS**
**49330 Chateauneuf-Sur-Sarthe (FR)**

(72) Inventeurs:
• **Hayne-Lecocq, Bénédicte**
  **75014 Paris (FR)**
• **Tebec, Jean-Louis**
  **91190 Gif Sur Yvette (FR)**
• **Van Ceulen, Vincent**
  **49100 Angers (FR)**

(74) Mandataire: **Coralis Harle**
**14-16 Rue Ballu**
**75009 Paris (FR)**

(54) **Système pour la gestion de l'état sanitaire et respiratoire de bovins**

(57)    La présente invention concerne un système pour la gestion de l'état sanitaire et respiratoire de bovins, ledit système de gestion (1) étant destiné à équiper une enceinte (E) d'élevage pour bovins.

Ce système de gestion 1 comprend :
i) au moins un dispositif de type microphone (2), pour l'acquisition de données sonores ambiantes au sein de ladite enceinte d'élevage (E),
ii) un dispositif de monitorage (3) comprenant :
- des moyens de traitement des données sonores ambiantes acquises, de sorte à détecter les évènements sonores correspondant à des toux,
- des moyens de comptage du nombre de toux sur une période de temps donnée, pour obtenir une valeur acquise de nombre d'évènements sonores détectés,
- des moyens de comparaison de ladite valeur acquise par rapport à une valeur seuil, et
- des moyens d'alerte d'un opérateur si ladite valeur acquise est supérieure à ladite valeur seuil.

Fig.1

EP 2 783 629 A1

## Description

DOMAINE TECHNIQUE AUQUEL SE RAPPORTE L'INVENTION

La présente invention concerne le domaine de l'élevage des bovins. Elle concerne plus particulièrement un système pour la gestion de l'état sanitaire et respiratoire de bovins, adapté pour équiper une enceinte d'élevage.

ARRIERE-PLAN TECHNOLOGIQUE

Les principaux facteurs affectant les performances d'un élevage de bovins sont liés au système digestif, au système respiratoire, et dans une moindre mesure au système de reproduction.

Les troubles respiratoires, notamment les toux, constituent des prémices de pathologies ; les anticiper permettrait de prévenir l'apparition et la propagation d'agents pathogènes susceptibles d'affecter le bien être de l'élevage.

A ce jour, l'apparition de ces troubles respiratoires est évaluée par l'éleveur, avec la part de subjectivité qui en découle.

Il s'agit par conséquent de passer d'une approche d'« appréciation » de la qualité respiratoire à une approche « objective » de l'état sanitaire et respiratoire des bovins, basée sur des critères scientifiques afin de maitriser efficacement le confort respiratoire des bovins au sein d'un élevage.

Des systèmes de gestion de ce genre sont connus pour les élevages de porc. Par exemple, le document WO-2008/152433 décrit succinctement une méthode d'analyse acoustique permettant de reconnaître des toux, et ensuite de localiser les sujets malades dans les enclos.

Cependant, les systèmes de gestion correspondants sont en pratique complexes et onéreux. De plus, l'analyse acoustique utilisée chez le porc n'est pas transposable directement à l'analyse acoustique chez les bovins du fait des adaptations nécessaires pour la détection des toux de ces animaux et des bruits ambiants spécifiques.

Il existe donc un besoin pour un système de gestion de l'état sanitaire et respiratoire de bovins, destiné en particulier à la surveillance de l'apparition des toux au sein d'un élevage et de leur mesure quantitative, exploitable in situ.

OBJET DE L'INVENTION

Dans ce contexte, le demandeur propose un système particulier pour la gestion de l'état sanitaire et respiratoire qui est dédié aux bovins, destiné à équiper une enceinte d'élevage pour ces animaux.

Ce système est caractérisé par le fait qu'il comprend :

i) au moins un dispositif de type microphone, pour l'acquisition de données sonores ambiantes au sein de ladite enceinte d'élevage,
ii) un dispositif de monitorage comprenant :

-    des moyens de traitement des données sonores ambiantes acquises, de sorte à détecter les événements sonores correspondant à des toux émises par les bovins de l'élevage,
-    des moyens de comptage du nombre desdits événements sonores détectés sur une période de temps donnée, pour obtenir une valeur acquise de nombre d'événements sonores détectés,
-    des moyens de comparaison de ladite valeur acquise de nombre d'évènements sonores détectés par rapport à une valeur seuil de nombre d'événements sonores, et
-    des moyens d'alerte d'un opérateur si ladite valeur acquise de nombre d'événements sonores est supérieure à ladite valeur seuil de nombre d'évènements sonores.

Afin de distinguer les événements sonores correspondant des toux par rapport aux événements sonores « parasites », les moyens de traitement des données sonores ambiantes acquises, comprennent avantageusement :

-    des moyens de filtrage fréquentiel desdites données sonores ambiantes acquises, pour éliminer le bruit de fond et pour générer des données sonores ambiantes acquises filtrées,
-    des moyens de seuillage desdites données sonores ambiantes acquises filtrées comprenant un seuil pour détecter, parmi les données sonores ambiantes acquises filtrées, toutes les émergences sonores dites « signaux émergeants » dépassant un seuil prédéterminé qui sont susceptibles de correspondre à une toux,
-    des moyens de calcul (i) de l'enveloppe sonore desdits signaux émergeants de sorte à déterminer la durée de chacun desdits signaux émergeants et (ii) le barycentre (en particulier la fréquence barycentrique) de chacun desdits signaux émergeants, et
-    des moyens de filtrage temporel et barycentrique (avantageusement fréquentiel), pour déterminer les signaux émergeants correspondant à une toux, tenant compte de domaines prédéterminés de durée et de barycentre (en particulier de fréquence barycentrique).

De manière inattendue, le couple de paramètres durée / fréquence barycentrique des signaux émergeants s'avère particulièrement efficace pour discriminer les événements sonores correspondant à une toux chez les bovins par rapport aux événements sonores autres que les toux de bovins.

Dans ce cas, les moyens de filtrage fréquentiel comprennent avantageusement un filtrage passe-bande des données sonores ambiantes acquises tenant compte de fréquences de coupure, d'un type de filtre et d'un ordre du filtre prédéterminés.

De préférence, pour les moyens de filtrage fréquentiel, les fréquences de coupure sont comprises entre 1 000 et 10 000 Hz, et l'ordre du filtre passe-bande est d'au moins 6.

De préférence également, pour les moyens de filtrage temporel et barycentrique :

- le domaine prédéterminé de durée est compris entre 0,1 s et 0,5 s, de préférence entre 0,18 s et 0,42 s, et
- le domaine prédéterminé de fréquence barycentrique est compris entre 2 000 et 5 000 Hz, de préférence entre 2 380 Hz et 3 630 Hz, de préférence encore entre 2 383 Hz et 3 621 Hz.

Par « filtrage barycentrique », on entend en particulier un filtrage fréquentiel.

Selon un mode de réalisation préféré, le dispositif de monitorage comprend des moyens de détection du niveau sonore ambiant, de sorte à autoriser la mise en oeuvre des moyens de traitement des données sonores ambiantes acquises seulement lorsque le niveau sonore ambiant est inférieur à un niveau sonore maximal prédéterminé.

Ce système de gestion comprend avantageusement au moins deux dispositifs de type microphones, pour obtenir au moins deux collectes de données sonores ambiantes provenant chacune d'une zone de ladite enceinte d'élevage.

Dans ce cas :

- les moyens fonctionnels du dispositif de monitorage sont mis en oeuvre sur chacune desdites données sonores ambiantes indépendamment l'une de l'autre, ou
- les moyens fonctionnels du dispositif de monitorage sont mis en oeuvre sur les données sonores ambiantes après leur combinaison.

Les moyens d'alerte comprennent avantageusement des moyens pour l'émission d'un message de préconisation d'au moins un additif alimentaire ou d'au moins un aliment complémentaire choisi parmi ceux aptes à favoriser le confort respiratoire des bovins.

En outre, le ou les dispositifs de type microphones sont avantageusement montés sur des supports munis de moyens de solidarisation dans l'enceinte d'élevage.

La présente invention concerne également le dispositif de monitorage en tant que tel, et aussi l'application logicielle pour ce dispositif de monitorage.

L'invention concerne encore l'enceinte d'élevage pour bovins, équipée d'un système de gestion selon l'invention.

La présente invention concerne encore un procédé pour la gestion de l'état sanitaire et respiratoire de bovins, lesdits bovins étant présents au moins une partie du temps au sein d'une enceinte d'élevage.

Ce procédé comprend les étapes suivantes :

(a) une étape d'acquisition, au sein de ladite enceinte d'élevage, de données sonores ambiantes par au moins un dispositif du type microphone,
(b) une étape de traitement des données sonores ambiantes acquises par un dispositif de monitorage, de sorte à détecter les événements sonores correspondant à des toux émises par les bovins de l'élevage,
(c) une étape de comptage, par ledit dispositif de monitorage, du nombre desdits événements sonores détectés sur une période de temps donnée, pour obtenir une valeur acquise de nombre d'événements sonores détectés,
(d) une étape de comparaison, par ledit dispositif de monitorage, de ladite valeur acquise de nombre d'évènements sonores détectés par rapport à une valeur seuil de nombre d'événements sonores, et
(e) une étape d'alerte d'un opérateur par ledit dispositif de monitorage, si ladite valeur acquise de nombre d'évènements sonores est supérieure à ladite valeur seuil de nombre d'événements sonores.

L'étape (b) de traitement des données sonores ambiantes acquises, comprend avantageusement :

(b1) une opération de filtrage fréquentiel desdites données sonores ambiantes acquises, pour éliminer le bruit de fond et pour générer des données sonores ambiantes acquises filtrées,

(b2) une opération de seuillage desdites données sonores ambiantes acquises filtrées comprenant un seuil pour détecter, parmi les données sonores ambiantes acquises filtrées, toutes les émergences sonores dépassant un seuil prédéterminé qui sont susceptibles de correspondre à une toux, dites « signaux émergeants »,

(b3) une opération de calcul (i) de l'enveloppe sonore desdits signaux émergeants de sorte à déterminer la durée de chacun desdits signaux émergeants et (ii) le barycentre (en particulier la fréquence barycentrique) de chacun desdits signaux émergeants, et

(b4) une opération de filtrage temporel et barycentrique (en particulier fréquentiel), pour déterminer les signaux émergeants correspondant à une toux, tenant compte de domaines prédéterminés de durée et de barycentre (de préférence de fréquence barycentrique).

Dans ce cas, l'opération (b1) de filtrage fréquentiel comprend un filtrage passe-bande des données sonores ambiantes acquises tenant compte de fréquences de coupure et d'un type de filtre et d'un ordre du filtre prédéterminés.

Lors de l'opération de filtrage fréquentiel (b1), les fréquences de coupure sont avantageusement comprises entre 1 000 et 10 000 Hz, et l'ordre du filtre passe-bande est d'au moins 6 ; le seuil de l'opération de seuillage (b2) est par exemple de 0,1.

Lors de l'opération (b4) de filtrage temporel et barycentrique (le filtrage barycentrique est avantageusement un filtrage fréquentiel), les domaines prédéterminés de durée et de fréquence barycentrique sont avantageusement respectivement les suivants :

- le domaine de durée est compris entre 0,1 s et 0,5 s, de préférence entre 0,18 s et 0,42 s, et
- le domaine de fréquence barycentrique est compris entre 2 000 et 5 000 Hz, de préférence entre 2 380 Hz et 3 630 Hz, de préférence encore entre 2 383 Hz et 3 621 Hz.

Préalablement à l'étape (a) d'acquisition de données sonores ambiantes, une étape de détection du niveau sonore ambiant est avantageusement mise en oeuvre, de sorte à autoriser ladite étape (a) d'acquisition de données sonores ambiantes seulement lorsque le niveau sonore ambiant est inférieur à un niveau sonore maximal prédéterminé.

L'étape (a) d'acquisition de données sonores ambiantes comprend éventuellement au moins deux collectes de données sonores ambiantes provenant chacune d'une zone de ladite enceinte d'élevage.

L'étape (e) d'alerte comprend avantageusement une opération de préconisation d'au moins un additif alimentaire ou d'au moins un aliment complémentaire choisi parmi ceux aptes à favoriser le confort respiratoire des bovins.

L'étape (e) d'alerte est de préférence suivie d'une étape (f) de distribution aux bovins dudit élevage d'au moins un additif alimentaire ou d'un aliment complémentaire choisi parmi ceux aptes à favoriser leur confort respiratoire.

Selon un mode de réalisation particulier, l'étape d'acquisition de données sonores ambiantes est mise en oeuvre par au moins deux dispositifs du type microphone, et les étapes suivantes sont mises en oeuvre sur : - chacune desdites données sonores ambiantes, indépendamment l'une de l'autre, et/ou - les données sonores ambiantes après leur addition.

L'invention concerne encore un programme d'ordinateur comprenant des moyens de programme pour l'exécution des étapes du procédé selon l'invention, lorsque ledit programme d'ordinateur est exécuté sur un ordinateur.

DESCRIPTION DETAILLEE DE L'INVENTION

La présente invention sera encore illustrée, sans être aucunement limitée par la description suivante d'un mode de réalisation particulier, cela en relation avec les dessins annexés dans lesquels :

- la figure 1 est une vue générale et schématique d'un système de gestion selon l'invention, équipant une enceinte d'élevage pour bovins ;
- la figure 2 représente, sous forme d'un schéma-bloc, les moyens fonctionnels constitutifs du dispositif de monitorage appartenant au système de gestion selon l'invention ;
- la figure 3 représente, également sous forme d'un schéma-bloc, les différents moyens de l'un des moyens fonctionnels constitutifs du dispositif de monitorage, à savoir les moyens de traitement des données sonores acquises.

Le système de gestion 1 selon l'invention, pour le monitorage de l'état sanitaire et respiratoire de bovins, est destiné à équiper une enceinte d'élevage E de ces animaux.

Par « enceinte d'élevage », on entend notamment une étable, c'est-à-dire la partie du bâtiment réservée à l'élevage des bovins.

Par « bovins », on entend les bovins de tous âges et de toutes races, à savoir notamment les veaux, les vaches notamment laitières, les génisses, les vaches taries, les bovins viandes ou les bovins à l'engrais.

Cette étable est donc le lieu où le bétail est mis en stabulation entravée ou libre.

Pour le monitorage de ces bovins, le système de gestion 1 selon l'invention comprend :

- des dispositifs de type microphones 2, pour l'acquisition de données sonores ambiantes au sein de l'enceinte d'élevage E, et
- un dispositif de monitorage 3, permettant (i) une mesure quantitative des toux émises par les bovins de l'élevage, partant des données sonores ambiantes acquises, et (ii) l'alerte d'un opérateur si le nombre de toux dépasse une valeur seuil prédéterminée (éventuellement calculée en fonction notamment du nombre d'animaux de l'élevage, voire même ajustable à façon).

Les dispositifs de type microphones 2 sont montés sur des supports, en hauteur dans l'enceinte d'élevage E, au-dessus des bovins.

Les supports (non représentés) sont munis de moyens de solidarisation (non représentés) dans l'enceinte d'élevage E, par exemple avec des éléments de structure ou de charpente de cette enceinte d'élevage E.

Ces microphones 2 se situent avantageusement à une hauteur de l'ordre de 2 à 6 mètres, par exemple de l'ordre de 3 m, par rapport au sol.

Ces microphones 2 sont avantageusement éloignés d'au moins 10 m, de préférence d'au moins 20 m, les uns des autres.

Un dispositif électronique du type « table de mixage » 15 est ici interposé entre, d'une part, les microphones 2 et, d'autre part, le dispositif de monitorage 3.

La table de mixage 15, classique en elle-même, est destinée à amplifier et sommer les données sonores ambiantes captées/collectées par les différents microphones 2. Une fois les données sonores ambiantes acquises « sommées », cette table de mixage 15 est en plus destinée à transmettre ces données au dispositif de monitorage 3.

De manière alternative, les données sonores ambiantes acquises par chaque microphone 2 peuvent être transmises, indépendamment les unes des autres, au dispositif de monitorage 3 de manière à permettre leur analyse acoustique ultérieure indépendamment les unes des autres.

Le dispositif de monitorage 3 consiste quant à lui en un dispositif électronique et/ou informatique apte à assurer une analyse acoustique de données sonores.

Ce dispositif de monitorage 3 consiste ainsi par exemple en un ordinateur ou en un micro-ordinateur, muni d'une unité de traitement de l'information, essentiellement un microprocesseur.

Différents moyens fonctionnels sont prévus au sein de ce dispositif de monitorage 3, avantageusement sous la forme d'un ou plusieurs programmes d'ordinateur 4 (dit encore « application(s) logicielle(s) », « logiciel » ou « programme informatique »).

Le ou les programmes d'ordinateur 4 comprennent des moyens de programme pour l'exécution des étapes du procédé selon l'invention, lorsque ledit programme d'ordinateur est exécuté sur un ordinateur.

Par « moyens de programme », on entend en particulier des portions ou des instructions de code de programme, lisibles par un ordinateur et destinés à être exécutés sur ledit ordinateur.

Ces différents moyens fonctionnels peuvent encore se présenter sous une forme électrique, analogique ou numérique.

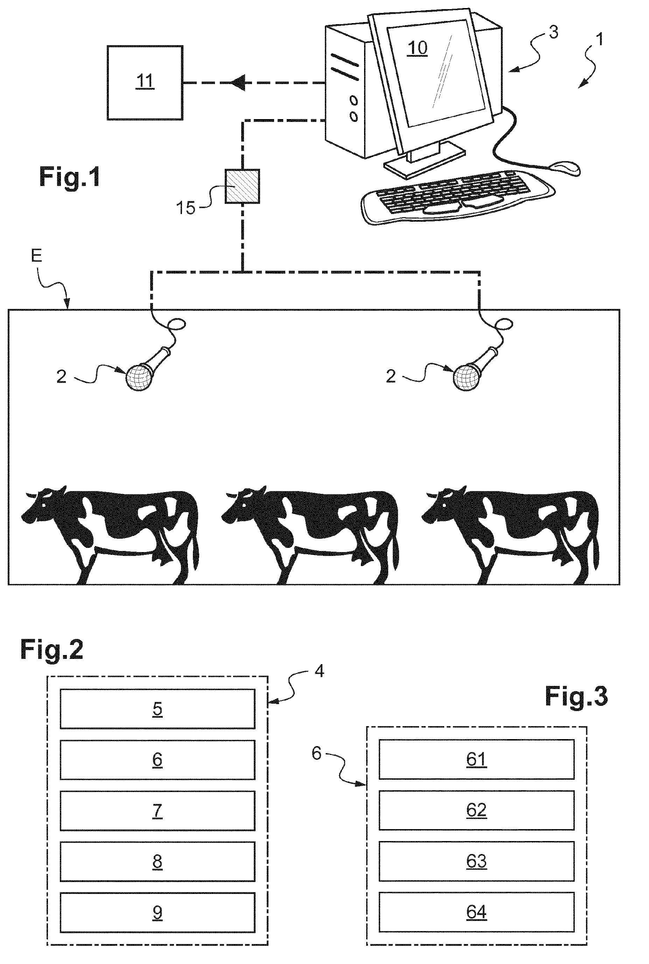

Tels qu'illustrés schématiquement sur la figure 2, ces moyens fonctionnels consistent en :

- des moyens 5 de détection du niveau sonore ambiant, de sorte à autoriser la mise en oeuvre des autres moyens du dispositif de monitorage 3 seulement lorsque le niveau sonore ambiant est inférieur à un niveau sonore maximal prédéterminé ;
- des moyens 6 de traitement des données sonores ambiantes acquises par les microphones 2, de sorte à détecter les événements sonores correspondant à des toux émises par les bovins de l'élevage,
- des moyens 7 de comptage du nombre desdits évènements sonores détectés sur une période de temps donnée, pour obtenir une valeur acquise de nombre d'évènements sonores détectés,
- des moyens 8 de comparaison de la valeur acquise de nombre d'évènements sonores détectés par rapport à une valeur seuil de nombre d'évènements sonores, et
- des moyens 9 d'alerte d'un opérateur si la valeur acquise de nombre d'évènements sonores est supérieure à la valeur seuil de nombre d'évènements sonores.

Le dispositif de monitorage 3 effectue régulièrement ou en continu des analyses sur des plages de 24 heures, avec par exemple, des prises d'enregistrement en continu et en temps réel sur des périodes comprises chacune entre 10 minutes et 60 minutes, de préférence de 30 minutes.

De manière générale, la durée des périodes d'enregistrement est adaptée à façon.

L'acquisition de ces périodes d'enregistrement s'effectue avantageusement à plusieurs reprises réparties sur la journée, qui sont représentatives de cette journée.

Dans l'enceinte E, les nuisances sonores liées à certains équipements (par exemple les ventilateurs) peuvent être importantes ; il est alors difficile d'effectuer une analyse sonore fiable des données sonores ambiantes.

Les moyens 5 de détection de niveau sonore ambiant permettent alors le lancement du traitement des données sonores ambiantes seulement lorsque le niveau sonore ambiant descend en-dessous d'un niveau sonore maximal prédéterminé.

En pratique, l'enregistrement et le traitement des bruits ambiants s'effectuent alors uniquement lorsque le niveau sonore des ventilateurs, ou des autres bruits masquants, est en dessous d'un seuil acceptable pour le monitorage respiratoire.

Là encore, ce seuil de niveau sonore est adapté à façon, notamment en fonction des bruits habituels de l'enceinte E.

Par exemple, ce niveau sonore maximal prédéterminé sera de 80 dB.

Les moyens 6 de traitement des données sonores ambiantes acquises, comprennent un programme d'ordinateur ou une combinaison de programmes d'ordinateur (ou une application logicielle, ou une combinaison d'applications logicielles) qui, lorsqu'il est exécuté par un ordinateur, est apte à effectuer un traitement et une analyse de fichiers sonores.

Ces moyens de traitement 6 peuvent encore se présenter sous une forme électrique, analogique ou numérique.

Ces moyens de traitement 6 permettent en particulier, d'une part, d'éliminer les bruits de fond présents dans l'enceinte E d'élevage de bovins et, d'autre part, de détecter au mieux les évènements sonores correspondants à des toux.

Ces bruits de fond se composent généralement :

- de cliquetis,
- des bruits extérieurs (camions, chantier, humains, oiseaux),
- des bruits de bovin lorsqu'il boit et se nourrit,
- du bruit vocal des bovins,
- des ventilateurs,
- de l'agitation très forte au moment du repas, ou encore
- du frottement des cornes ou des pattes contre les structures de l'enceinte E.

Tel qu'illustré sur la figure 3, ces moyens de traitement 6 sont programmés pour constituer :

- des moyens 61 de filtrage fréquentiel des données sonores ambiantes acquises, pour éliminer le bruit de fond et pour générer des données sonores ambiantes acquises filtrées,
- des moyens 62 de seuillage desdites données sonores ambiantes acquises filtrées, comprenant un seuil pour détecter, parmi les données sonores ambiantes acquises filtrées, toutes les émergences sonores dépassant un seuil prédéterminé qui sont susceptibles de correspondre à une toux, c'est-à-dire des « signaux émergeants »,
- des moyens 63 de calcul (i) de l'enveloppe sonore desdits signaux émergeants de sorte à déterminer la durée desdits signaux émergeants et (ii) d'un paramètre du barycentre, en l'occurrence de la fréquence barycentrique, de chacun desdits signaux émergeants, et
- des moyens 64 de filtrage temporel et fréquentiel, pour déterminer les signaux émergeants correspondant à une toux, tenant compte de domaines prédéterminés de durée et de fréquence barycentrique.

Les moyens 61 de filtrage fréquentiel comprennent un filtrage passe-bande des données sonores ambiantes acquises.

Par « filtre passe-bande », on entend un filtre ne laissant passer les signaux que dans un intervalle de fréquences compris entre une fréquence de coupure basse et une fréquence de coupure haute.

Pour réduire efficacement le bruit de fond, il est possible d'ajuster la valeur de différents paramètres prédéterminés de ce filtre passe-bande : les fréquences de coupure, le type de filtre et l'ordre de filtre.

Par « fréquence de coupure », on entend les fréquences maximales et minimales, en deçà et au-delà desquelles les données sonores sont traitées.

En d'autres termes, cette fréquence de coupure correspond à la fréquence pour laquelle le facteur de transmission d'un filtre est égal à une valeur donnée, généralement -3 décibels (correspondant à 0,707).

La réduction de la largeur du filtre passe-bande, correspondant à la « fréquence de coupure », permet une élimination du bruit de fond plus importante.

Ces fréquences de coupure sont avantageusement comprises entre 1 000 Hz et 10 000 Hz.

Le type de filtre est quant à lui avantageusement choisi parmi un filtre connu, par exemple le filtre de Bessel, le filtre de Butterworth ou le filtre de Tchebychev.

Par « ordre du filtre », on entend le degré de la fonction rationnelle dont le numérateur et le dénominateur sont constitués par un polynôme représentant la fonction de transfert du filtre. La raideur de la pente d'atténuation dans la zone de transition est d'autant plus importante que l'ordre du filtre est élevé.

De préférence, l'ordre du filtre est égal, ou supérieur, à 6.

En l'occurrence, le filtre passe-bande est avantageusement de type Tchebychev ordre 6 de type 1.

Les moyens de seuillage 62 consistent quant à eux à déterminer les événements sonores dont l'amplitude atteint ou dépasse un niveau sonore sélectionné, ces émergences sonores étant susceptibles de correspondre à une toux, en vue de leur analyse à venir.

Le seuil prédéterminé des moyens de seuillage 62 est ainsi sélectionné de sorte à éliminer les signaux dont l'amplitude est inférieure à un niveau sélectionné, correspondant à niveaux sonores faibles (pour éliminer par exemple les bruissements, les bruits éloignés, etc.).

Partant des signaux émergeants, les moyens de calcul 63 sont mis en oeuvre pour déterminer l'enveloppe sonore des signaux émergeants.

Par « enveloppe sonore », on entend l'évolution de son amplitude (son volume) au cours du temps.

Elle peut être représentée par une courbe qui présente trois parties : l'attaque (segment ascendant de la courbe), le maintien (partie supérieure de la cloche) et la chute (segment descendant).

Pour lisser la forme de l'enveloppe obtenue, le calcul des enveloppes sonores peut être réalisé. Ce lissage est utile pour éviter les oscillations qui fausseraient la mesure de durée des événements.

Partant de ces enveloppes sonores lissées, les moyens de calcul 63 déterminent la durée de chacun des signaux émergeants.

Ces moyens de calcul 63 déterminent également un paramètre lié au barycentre, en l'occurrence le barycentre spectral.

Par « barycentre spectral », on entend avantageusement le barycentre des fréquences ou la fréquence barycentrique précitée.

Cette fréquence barycentrique peut être obtenue par la formule suivante :

$$\text{Fréquence barycentrique (Hz)} = \Sigma(\text{Amplitude spectrale} \times \text{fréquence}) / \Sigma\text{Amplitude spectrale}$$

La durée de chacun desdits signaux émergeants est choisie avec un seuil d'amplitude visant à éviter les artéfacts.

Les moyens 64 de filtrage temporel et fréquentiel vont ensuite permettre de déterminer les signaux émergeants correspondant à une toux, tenant compte de domaines prédéterminés suivants :

- un domaine de durée compris entre 0,1 seconde et 0,5 seconde, de préférence entre 0,18 et 0,42 seconde, et
- un domaine de fréquence barycentrique avantageusement compris entre 2 000 et 5 000 Hz, de préférence entre 2380 Hz et 3630 Hz, de préférence encore entre 2383 Hz et 3621 Hz.

Les signaux émergeants dont la durée et la fréquence barycentrique sont contenues dans ces deux domaines prédéterminés correspondent à une toux chez les bovins.

Le demandeur a constaté que le couplage de ces deux critères permet une détection optimale des toux de bovins.

En pratique, ces domaines appliqués par les moyens de filtrage 64 peuvent être affinés afin d'isoler au mieux les événements sonores correspondants à des toux, par rapport aux autres bruits survenant dans l'enceinte (par exemple les bruits de ferraille).

Ces domaines prédéterminés peuvent être modifiés à distance, notamment via une interface réseau (s'appuyant par exemple sur un réseau Internet).

Des essais réalisés dans différentes enceintes d'élevage donnent une efficacité de détection des toux jusqu'à 98 %.

Partant des événements sonores détectés comme étant des toux émises par les bovins, les moyens de comptage 7 déterminent le nombre de ces événements sonores sur une période de temps donnée, pour obtenir une valeur acquise de nombre d'événements sonores détectés.

Par exemple, cette détermination du nombre d'événements sonores s'effectue sur des périodes de 10 à 60 min, de préférence de 25 à 35 min, de préférence encore de 30 min.

Par des « périodes de 10 à 60 min », on entend encore les périodes de 10, 15, 20, 25, 30, 35, 40, 45, 50, 55 et 60 min.

La valeur acquise est avantageusement extrapolée ensuite selon un nombre de toux par animal et par période de temps (par heure par exemple).

Les moyens de comparaison 8 sont quant à eux destinés à mettre en correspondance cette valeur acquise du nombre d'événements sonores détectés par rapport à la valeur seuil du nombre d'événements sonores.

Par exemple, pour des bovins, cette valeur seuil est de 5 à 15 toux par animal et par heure (de préférence encore de 10 toux par animal et par heure).

Dans le cas où la valeur acquise est supérieure à la valeur seuil, les moyens d'alerte 9 sont activés.

Ces moyens d'alerte 9 comprennent des moyens pour l'émission d'un message de préconisation pour un opérateur, en particulier l'éleveur.

En pratique, tel qu'illustré sur la figure 1, ce message de préconisation peut apparaître directement sur des moyens d'affichage 10 portés par le dispositif de monitorage 3 ou bien être transmis jusqu'à un dispositif électronique portatif 11, tel qu'un téléphone portable, une tablette numérique ou un ordinateur portable.

Toujours pour optimiser ce système de gestion, ce message de préconisation peut préciser à l'opérateur au moins un additif alimentaire, ou au moins un aliment complémentaire, à distribuer aux animaux, choisi parmi ceux aptes à favoriser le confort respiratoire des bovins.

Par « additif alimentaire », on entend en particulier une substance intentionnellement ajoutée dans le procédé de fabrication d'un aliment. Cet additif alimentaire consiste en un produit naturel ou un produit synthétisé.

Ces additifs alimentaires font en particulier l'objet d'une réglementation en Europe sous la référence CE n°1831/2003 du Parlement et du Conseil.

Par « aliment complémentaire », on entend en particulier tout aliment composé qui ne permet pas à lui seul la ration journalière d'un animal et peut, ou pas, comporter des additifs.

Ces aliments complémentaires font en particulier l'objet d'une réglementation en Europe sous la référence CE n°767/2009 du Parlement et du Conseil.

Ces aliments complémentaires peuvent être choisis par exemple parmi PREMEL 35 BKP, RESPIRAL ou VENTAL, commercialisés par la société NUTRAL SAS (CHATEAUNEUF-SUR-SARTHE - France).

De manière avantageuse, le système de gestion 1 est raccordé à un réseau général d'alerte (non représenté).

Ainsi chaque système de gestion 1 implanté pourra transmettre, de manière régulière, un rapport contenant le nombre d'événements sonores détectés et les messages d'alerte émis.

En retour, ce réseau général d'alerte pourra émettre des messages d'informations ciblés à destination des opérateurs pour leur faire part des évolutions épidémiologiques sur l'état sanitaire et respiratoire des bovins, tenant compte notamment d'indications géographiques.

En fonctionnement, le système de gestion 1 selon l'invention permet une surveillance de l'état sanitaire et respiratoire de bovins séjournant au moins une partie du temps au sein de l'enceinte d'élevage E.

Ce système de gestion 1 effectue des analyses acoustiques de manière régulière sur des plages de 24 heures avec, par exemple, des prises d'enregistrements en temps réel de 30 minutes.

Le procédé mis en oeuvre comprend les étapes suivantes :

(a) une étape d'acquisition, au sein de ladite enceinte d'élevage E, de données sonores ambiantes par les microphones 2,
(b) une étape de traitement des données sonores ambiantes acquises par le dispositif de monitorage 3, de sorte à détecter les événements sonores correspondant à des toux émises par les bovins de l'élevage, comprenant avantageusement les opérations successives suivantes :

(b1) une opération de filtrage fréquentiel desdites données sonores ambiantes acquises (via les moyens de filtrage 61), pour générer les données sonores ambiantes acquises filtrées,
(b2) une opération de seuillage desdites données sonores ambiantes acquises filtrées (via les moyens de seuillage 62) comprenant le seuil pour détecter, parmi les données sonores ambiantes acquises filtrées, toutes les émergences sonores dépassant le seuil prédéterminé qui sont susceptibles de correspondre à une toux,
(b3) une opération de calcul de deux données, via les moyens de calcul 63, à savoir (i) l'enveloppe sonore desdits signaux émergeants de sorte à déterminer la durée de chacun desdits signaux émergeants et (ii) la fréquence barycentrique de chacun desdits signaux émergeants,
(b4) une opération de filtrage temporel et fréquentiel (via les moyens de filtrage dédiés 64), pour déterminer les signaux émergeants correspondant à une toux, tenant compte des domaines prédéterminés de durée et de fréquence barycentrique.

(c) une étape de comptage, par ledit dispositif de monitorage 3 (en particulier par la mise en oeuvre de ses moyens de comptage dédiés 7), du nombre desdits événements sonores détectés sur une période de temps donnée, pour obtenir une valeur acquise de nombre d'événements sonores détectés,
(d) une étape de comparaison, par ledit dispositif de monitorage 3 (en particulier par la mise en oeuvre de ses moyens de comparaison dédiés 8), de ladite valeur acquise de nombre d'événements sonores détectés par rapport

**EP 2 783 629 A1**

à une valeur seuil de nombre d'événements sonores, et

(e) une étape d'alerte d'un opérateur par ledit dispositif de monitorage 3 (en particulier via ses moyens d'alerte 9), si ladite valeur acquise de nombre d'évènements sonores est supérieure à ladite valeur seuil de nombre d'événements sonores.

Dans le cas où le système de gestion 1 identifie un nombre anormal de toux chez les bovins de l'élevage, c'est-à-dire encore un nombre de toux détectées dont la valeur est supérieure par rapport à une valeur seuil de nombre de toux, ce système de gestion 1 émet alors un message d'alerte à destination de l'opérateur.

Cette alerte peut être globale pour l'ensemble des animaux de l'élevage ; mais elle peut également être accompagnée d'une indication de localisation dans l'enceinte E, si le système de gestion 1 est configuré avec plusieurs microphones 2 dont les signaux sonores respectifs sont analysés indépendamment.

Cette alerte peut être accompagnée de la préconisation nutritionnelle précitée.

S'il le souhaite, l'opérateur peut alors incorporer le ou les additifs alimentaires et/ou le ou les aliments complémentaires appropriés dans les rations alimentaires des animaux.

L'invention permet ainsi :

- un enregistrement régulier et objectif du nombre d'événements sonores correspondant à des toux,
- un traitement de l'information suivant les seuils qui seront définis en collaboration avec les personnes compétentes (notamment des vétérinaires et des éleveurs),
- un avertissement à distance de l'éleveur afin qu'il puisse mener correctement sa conduite d'élevage, notamment en lui indiquant éventuellement la zone dans les lots définis comme « malades », et en lui préconisant les actions à conduire suivant l'intensité quantitative des toux enregistrées,
- une préconisation des gestes simples pour améliorer la gestion des lots en fonction de l'alimentation donnée ou de l'arrivée des animaux dans le bâtiment.

**Revendications**

1. Système pour la gestion de l'état sanitaire et respiratoire de bovins, ledit système de gestion (1) étant destiné à équiper une enceinte (E) d'élevage pour bovins, **caractérisé en ce qu'**il comprend :

   i) au moins un dispositif de type microphone (2), pour l'acquisition de données sonores ambiantes au sein de ladite enceinte d'élevage (E),
   ii) un dispositif de monitorage (3) comprenant :

   - des moyens (6) de traitement des données sonores ambiantes acquises, de sorte à détecter les événements sonores correspondant à des toux émises par les bovins de l'élevage,
   - des moyens (7) de comptage du nombre desdits événements sonores détectés sur une période de temps donnée, pour obtenir une valeur acquise de nombre d'événements sonores détectés,
   - des moyens (8) de comparaison de ladite valeur acquise de nombre d'événements sonores détectés par rapport à une valeur seuil de nombre d'évènements sonores, et
   - des moyens (9) d'alerte d'un opérateur si ladite valeur acquise de nombre d'événements sonores est supérieure à ladite valeur seuil de nombre d'évènements sonores.

2. Système selon la revendication 1, **caractérisé en ce que** les moyens (6) de traitement des données sonores ambiantes acquises, comprennent :

   - des moyens (61) de filtrage fréquentiel desdites données sonores ambiantes acquises, pour éliminer le bruit de fond et pour générer des données sonores ambiantes acquises filtrées,
   - des moyens (62) de seuillage desdites données sonores ambiantes acquises filtrées comprenant un seuil pour détecter, parmi les données sonores ambiantes acquises filtrées, tous les signaux émergeants dépassant un seuil prédéterminé qui sont susceptibles de correspondre à une toux,
   - des moyens (63) de calcul (i) de l'enveloppe sonore desdits signaux émergeants de sorte à déterminer la durée desdits signaux émergeants et (ii) du barycentre de chacun desdits signaux émergeants, et
   - des moyens (64) de filtrage temporel et barycentrique, pour déterminer les signaux émergeants correspondant à une toux, tenant compte de domaines prédéterminés de durée et de barycentre.

3. Système selon la revendication 2, **caractérisé en ce que** les moyens de filtrage fréquentiel (61) comprennent un filtrage passe-bande des données sonores ambiantes acquises tenant compte de fréquences de coupure, d'un type de filtre et d'un ordre du filtre prédéterminés.

4. Système selon la revendication 3, **caractérisé en ce que**, pour les moyens de filtrage fréquentiel (61), les fréquences de coupure sont comprises entre 1 000 et 10 000 Hz, et l'ordre du filtre passe-bande est d'au moins 6.

5. Système selon l'une quelconque des revendications 2 à 4, **caractérisé en ce que**, pour les moyens (64) de filtrage temporel et barycentrique du type fréquentiel, les domaines prédéterminés de durée et de fréquence barycentrique sont respectivement les suivants :

   - le domaine de durée est compris entre 0,1 s et 0,5 s, et
   - le domaine de fréquence barycentrique est compris entre 2 000 et 5 000 Hz.

6. Système selon l'une quelconque des revendications 1 à 5, **caractérisé en ce que** le dispositif de monitorage (3) comprend des moyens (5) de détection du niveau sonore ambiant, de sorte à autoriser la mise en oeuvre des moyens (6) de traitement des données sonores ambiantes acquises seulement lorsque le niveau sonore ambiant est inférieur à un niveau sonore maximal prédéterminé.

7. Système selon l'une quelconque des revendications 1 à 6, **caractérisé en ce qu'**il comprend au moins deux dispositifs de type microphones (2), pour obtenir au moins deux collectes de données sonores ambiantes provenant chacune d'une zone de ladite enceinte d'élevage (E), et **en ce que** :

   - les moyens (6, 7, 8, 9) du dispositif de monitorage (3) sont mis en oeuvre sur chacune desdites données sonores ambiantes indépendamment l'une de l'autre, ou
   - les moyens (6, 7, 8, 9) du dispositif de monitorage sont mis en oeuvre sur les données sonores ambiantes après leur addition.

8. Système selon l'une quelconque des revendications 1 à 7, **caractérisé en ce que** les moyens d'alerte (9) comprennent des moyens pour l'émission d'un message de préconisation d'au moins un additif alimentaire ou d'au moins un aliment complémentaire choisi parmi ceux aptes à favoriser le confort respiratoire des bovins.

9. Système selon l'une quelconque des revendications 1 à 8, **caractérisé en ce que** le ou les dispositifs de type microphones (2) sont montés sur des supports munis de moyens de solidarisation dans l'enceinte d'élevage (E).

10. Enceinte d'élevage pour bovins, **caractérisée en ce qu'**elle est équipée d'un système de gestion (1) selon l'une quelconque des revendications 1 à 9.

11. Dispositif de monitorage (3) pour un système de gestion (1) selon l'une quelconque des revendications 1 à 9, comprenant :

   - des moyens (6) de traitement des données sonores ambiantes acquises par au moins un dispositif de type microphone, de sorte à détecter les événements sonores correspondant à des toux émises par les bovins de l'élevage,
   - des moyens (7) de comptage du nombre desdits évènements sonores détectés sur une période de temps donnée, pour obtenir une valeur acquise de nombre d'événements sonores détectés,
   - des moyens (8) de comparaison de ladite valeur acquise de nombre d'évènements sonores détectés par rapport à une valeur seuil de nombre d'évènements sonores, et
   - des moyens (9) d'alerte d'un opérateur si ladite valeur acquise de nombre d'évènements sonores est supérieure à ladite valeur seuil de nombre d'évènements sonores.

12. Procédé pour la gestion de l'état sanitaire et respiratoire de bovins, lesdits bovins étant présents au moins une partie du temps au sein d'une enceinte d'élevage (E), **caractérisé en ce que** ledit procédé comprend les étapes suivantes :

   (a) une étape d'acquisition, au sein de ladite enceinte d'élevage (E), de données sonores ambiantes par la mise en oeuvre d'au moins un dispositif du type microphone (2),
   (b) une étape de traitement des données sonores ambiantes acquises par un dispositif de monitorage (3), de sorte à détecter les évènements sonores correspondant à des toux émises par les bovins de l'élevage,

(c) une étape de comptage du nombre desdits événements sonores détectés sur une période de temps donnée, par la mise en oeuvre dudit dispositif de monitorage (3), pour obtenir une valeur acquise de nombre d'événements sonores détectés,

(d) une étape de comparaison de ladite valeur acquise de nombre d'évènements sonores détectés par rapport à une valeur seuil de nombre d'évènements sonores, par la mise en oeuvre dudit dispositif de monitorage (3), et

(e) une étape d'alerte d'un opérateur par ledit dispositif de monitorage (3), si ladite valeur acquise de nombre d'évènements sonores est supérieure à ladite valeur seuil de nombre d'évènements sonores.

**13.** Procédé pour la gestion de l'état sanitaire et respiratoire de bovins selon la revendication 12, **caractérisé en ce que** l'étape (e) d'alerte comprend une opération de préconisation d'au moins un additif alimentaire ou d'au moins un aliment complémentaire choisi parmi ceux aptes à favoriser le confort respiratoire des bovins.

**14.** Procédé pour la gestion de l'état sanitaire et respiratoire de bovins selon l'une quelconque des revendications 12 ou 13, **caractérisé en ce que** l'étape d'acquisition de données sonores ambiantes est mise en oeuvre par au moins deux dispositifs du type microphone (2), et

**en ce que** les étapes suivantes dudit procédé de gestion sont mises en oeuvre sur :

- chacune desdites données sonores ambiantes, indépendamment l'une de l'autre, et/ou
- les données sonores ambiantes après leur addition.

**15.** Programme d'ordinateur comprenant des moyens de programme pour l'exécution des étapes du procédé selon l'une quelconque des revendications 12 à 14, lorsque ledit programme d'ordinateur est exécuté sur un ordinateur.

**Fig.1**

**Fig.2**

**Fig.3**

Europäisches
Patentamt

European
Patent Office

Office européen
des brevets

**RAPPORT DE RECHERCHE EUROPEENNE**

Numéro de la demande

EP 14 30 5455

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2007/276278 A1 (COYLE MICHAEL [US] ET AL) 29 novembre 2007 (2007-11-29) * abrégé * * alinéas [0009] - [0023], [0046] - [0067], [0083] - [0105]; figures 1-9 * | 1-15 | INV. A61B5/08 A61B7/00 |
| A | WO 2008/135985 A1 (EARLYSENSE LTD [IL]; HALPERIN AVNER [IL]; TSOREF LIAT [IL]; GROSS YOSE) 13 novembre 2008 (2008-11-13) * abrégé * * pages 55-70; figures 1-4 * | 1-15 | |
| A | JP 2007 135484 A (SHARP KK) 7 juin 2007 (2007-06-07) * abrégé * * Une traduction informatique automatisé de cette publication japonaise peut être consulté sur le site Web de l'Office japonais des brevets: http://ipdl.inpit.go.jp/homepg e.ipdl * | 1-15 | |
| A | EP 2 438 812 A1 (INC NAT UNIVERSITY IWATE UNIVERSITY [JP]) 11 avril 2012 (2012-04-11) * abrégé; figure 1 * | 1-15 | DOMAINES TECHNIQUES RECHERCHES (IPC) A61B |
| A | US 2012/302898 A1 (ZHANG XUSHENG [US] ET AL) 29 novembre 2012 (2012-11-29) * le document en entier * | 1-15 | |

Le présent rapport a été établi pour toutes les revendications

| Lieu de la recherche | Date d'achèvement de la recherche | Examinateur |
|---|---|---|
| La Haye | 26 mai 2014 | Juárez Colera, M |

**ANNEXE AU RAPPORT DE RECHERCHE EUROPEENNE
RELATIF A LA DEMANDE DE BREVET EUROPEEN NO.**

EP 14 30 5455

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

26-05-2014

| Document brevet cité au rapport de recherche | | Date de publication | Membre(s) de la famille de brevet(s) | | Date de publication |
|---|---|---|---|---|---|
| US 2007276278 | A1 | 29-11-2007 | US | 2007276278 A1 | 29-11-2007 |
| | | | WO | 2008131387 A2 | 30-10-2008 |
| WO 2008135985 | A1 | 13-11-2008 | EP | 2142095 A1 | 13-01-2010 |
| | | | US | 2008275349 A1 | 06-11-2008 |
| | | | US | 2012132211 A1 | 31-05-2012 |
| | | | WO | 2008135985 A1 | 13-11-2008 |
| JP 2007135484 | A | 07-06-2007 | JP | 4637728 B2 | 23-02-2011 |
| | | | JP | 2007135484 A | 07-06-2007 |
| EP 2438812 | A1 | 11-04-2012 | EP | 2438812 A1 | 11-04-2012 |
| | | | US | 2012088988 A1 | 12-04-2012 |
| | | | WO | 2010147175 A1 | 23-12-2010 |
| US 2012302898 | A1 | 29-11-2012 | AUCUN | | |

EPO FORM P0460

Pour tout renseignement concernant cette annexe : voir Journal Officiel de l'Office européen des brevets, No.12/82

## RÉFÉRENCES CITÉES DANS LA DESCRIPTION

**Documents brevets cités dans la description**

- WO 2008152433 A